# EUROPEAN PATENT SPECIFICATION

(11) **EP 0 887 404 B1**
(45) Date of publication and mention of the grant of the patent: **05.07.2006**
(21) Application number: 97906838.4
(22) Date of filing: 05.03.1997
(51) Int. Cl.: C12N 5/10, C12N 15/62, C07K 19/00, C07K 14/715, C07K 14/72

(54) **GENE THAT IMPARTS SELECTIVE PROLIFERATIVE ACTIVITY**
GEN FUER SELEKTIVE PROLIFERATION
GENE PROVOQUANT UNE PROLIFERATION SELECTIVE

(30) Priority: 05.03.1996 JP 847796
(43) Date of publication of application: 30.12.1998
(73) Proprietor: Dnavec Research Inc., Tsukuba-shi, Ibaraki 305 (JP)
(72) Inventor: OZAWA, Keiya, Kawachi-gun, Tochigi 329-04 (JP); ITOH, Katsuhisa, Tsukuba-shi, Ibaraki 305 (JP); SAKATA, Tsuneaki, Tsukuba-shi, Ibaraki 305 (JP); UEDA, Yasuji, Tsukuba-shi, Ibaraki 305 (JP); HASEGAWA, Mamoru, Tsukuba-shi, Ibaraki 305 (JP)
(74) Representative: Vossius & Partner
(86) International application number: PCT/JP1997/000687
(87) International publication number: WO 1997/032971

(56) References cited:
- CHAN C-H ET AL: "A MURINE CYTOKINE FUSION TOXIN SPECIFICALLY TARGETING THE MURINE GRANULOCYTE-MACROPHAGE COLONY-STIMULATING FACTOR (GM-CSF) RECEPTOR ON NORMAL COMMITED BONE MARROW PROGENITOR CELLS AND GM-CSF- DEPENDENT TUMOR CELLS" BLOOD, W.B. SAUNDERS, PHILADELPHIA, VA, US, vol. 86, no. 7, 1 October 1995 (1995-10-01), pages 2732-2740, XP000611396 ISSN: 0006-4971
- WILLIAMS D E ET AL: "HEMATOPOIETIC EFFECTS OF A GRANULOCYTE-MACROPHAGE COLONY-STIMULATING FACTOR INTERLEUKIN-3 FUSION PROTEIN" CANCER (PHILADELPHIA), vol. 67, no. 10 SUPPL., 1991, pages 2705-2707, XP002206890 ISSN: 0008-543X
- EMBO J., (1993), Vol. 12, No. 7, Jackson P. et al., "Hormone-Conditional Transformation by Fusion Proteins of c-Ab1 and Its Transforming Variants", p. 2809-2819. XP001084268
- PROG. GROWTH. FACTOR REF., (1991), Vol. 3, NAGATA S. et al., "Granulocyte Colony-Stimulating Factor and Its Receptor", p. 131-141. XP002961994
- BLOOD, (1996, Dec.), Vol. 88, ITO K. et al., "G-CSFR-Estrogen-R Fusion cDNA as a Novel Selective Amplifier Gene for Controllable Expansion of Transduced Hematopoietic Stem Cells", p. 137A. XP002962000
- EXPERIMENTAL HEMATOLOGY, (1996, Aug.), Vol. 24, No. 9, OZAWA K. et al., "Development of a Novel Selective Amplifier Gene For In Vivo Selective Expansion of Transduced Hematopoietic Stem Cells", p. 1053. XP002961995

## Description

### Technical Field

The present invention relates to the field of genetic engineering, particularly the field of gene therapy.

### Background Art

Various methods have so far been devised to treat diseases caused by congenital or acquired genetic defects, namely, gene disorders . In gene therapy, one such method, a defective gene itself is substituted by or supplemented with a normal gene in order to fundamentally cure gene disorders. It is important for the success of gene therapy to introduce a normal gene accurately into target cells and to express the introduced gene accurately. The conventionally used vectors for introducing a normal gene into target cells are viral vectors such as retrovirus vectors, adenovirus vectors, and adeno-associated virus vectors, and non-viral vectors such as liposomes. However, all have some shortcomings such as low gene introduction efficiency into target cells. Furthermore, they are often inadequate for treatment because of additional disadvantages such as poor expression efficiency of an introduced gene. In adenosine deaminase (ADA) deficiency, the cells into which the normal ADA gene has been introduced are expected to acquire a survival advantage or a growth advantage and gradually become dominant as a result of *in vivo* selection. In such a case, it may be possible to obtain gradual treatment effects despite the poor gene introduction efficiency. However, it is often necessary to introduce a gene for treatment that cannot be selected *i*n *vivo.* It has thus been desired to establish a system that enables selective amplification of cells containing an introduced gene.

Although G-CSF has been traditionally considered to be a cytokine (a hematopoietic factor) that selectively proliferates neutrophils, it has recently been reported that the administration of G-CSF increases not only neutrophils but also the hematopoietic stem cell /precursor cell pool in the body (Rinsho Ketsueki (Clinical Blood), 35, 1080 (1994)). The mechanism of manifestation of the G-CSF function has been reported to be dimerization of a G-CSF receptor that takes place upon activation of the G-CSF receptor by stimulation with G-CSF (Proc. Growth Factor Res., 3 (2), 131-141 (1991)). It has also been reported that the G-CSF receptor has a proliferation-inducing domain and a differentiation-inducing domain (Cell, 74, 1079-1087 (1993)). Moreover, like the G-CSF receptor, an estrogen receptor is known to be activated through dimerization (J Biol. Chem., 264, 2397-2400 (1989)), and there is a report that expression of a fusion protein between the estrogen receptor and c-Abl tyrosine kinase in the cell resulted in activation of the c-Abl tyrosine kinase (The EMBO Journal, 12, 2809-2819 (1993)).

### Disclosure of the Invention

The present invention seeks to overcome the problem of poor gene introduction efficiency by selectively amplifying *ex vivo* hematopoietic stem cells into which a gene for treatment has been introduced. The objective of the invention is to provide a fundamental technique for gene therapy targeting hematopoietic stem cells.

In the field of gene therapy today, there are numerous problems to be overcome concerning the efficiency of gene introduction into target cells and the expression efficiency of the introduced gene. It is therefore obvious that establishing a system for selectively amplifying only the target cells containing the introduced gene will produce a major breakthrough. In particular, if such a system is established for hematopoietic stem cells, which are the origin of many blood cells such as red blood cells or white blood cells and which are considered to be the most preferable target cells for gene therapy, it would contribute significantly to the field of gene therapy.

G-CSF, which was traditionally thought to be a cytokine (a hematopoietic factor) that selectively proliferates neutrophils, can also proliferate hematopoietic stem cells. The G-CSF receptor dimerizes itself when it is activated. Considering these facts, the present inventors have thought of a system for amplifying hematopoietic stem cells through dimerization of a genetically engineered G-CSF receptor. Also based on the fact that the estrogen receptor dimerizes itself upon stimulation with estrogen, the present inventors have thought of constructing a chimeric gene between the G-CSF receptor gene and the estrogen receptor gene, introducing the chimeric gene into cells, and externally stimulating the cells by estrogen to forcibly dimerize the G-CSF receptor portion of the chimeric gene product.

Thus, the present invention was completed by developing a new system for selectively amplifying hematopoietic stem cells into which a gene has been introduced by activating the G-CSF receptor portion of the chimeric gene product through external stimulation with estrogen, so as to apply this system to the field of gene therapy,

The present invention relates to a fusion protein as defined hereinafter; a vector comprising a gene encoding the fusion protein; a cell containing the vector; and a method for selectively proliferating the cell *ex vivo* by exposing the cell to a steroid hormone. Furthermore, when the vector contains an exogenous gene, the present invention relates to a method for selectively proliferating a cell into which the exogenous gene has been introduced.

More specifically, the present invention relates to:
(1) A fusion protein comprising
   (a) a first polypeptide and
   (b) a second polypeptide,
      wherein said first polypeptide comprises a ligand-binding domain of a steroid hormone receptor that seff-associates upon ligand binding, and wherein said second polypeptide comprises a cytokine receptor or a proliferation-inducing part thereof that, upon said self-association of said first polypeptide, imparts proliferation activity to a cell.
(2) The fusion protein of (1), wherein the steroid hormone receptor is an estrogen receptor.
(3) The fusion protein of (1), wherein the cytokine receptor is a G-CSF receptor.
(4) A vector comprising a gene encoding the fusion protein of (1).
(5) A cell carrying the vector of (4).
(6) An in vitro method for selectively proliferating the cell of 6 which comprises exposing the cell of (5) to a ligand capable of acting on the "ligand-binding domain" of the fusion protein of (1).
(7) A vector comprising a desired exogenous gene and a gene encoding the fusion protein according (1).
(8) The vector of (7), wherein the steroid hormone receptor is an estrogen receptor.
(9) The vector of (7), wherein the cytokine receptor is a G-CSF receptor.
(10) The vector of (7), wherein the "gene encoding a fusion protein" and the "exogenous gene" are located on the same molecule.
(11) The vector of (7), wherein the gene encoding a fusion protein" and the "exogenous gene" are located on separate molecules.
(12) A cell carrying the vector according to any one of (7) to (11).
(13) An in vitro method for selectively proliferating the cell of (12), which comprises exposing the cell of (12) to a ligand capable of acting on the "ligand-binding domain" of the fusion protein encoded by the gene contained in the vector of (7).
(14) A kit comprising (a) the vector of (4) or (7) and (b) a ligand capable of acting on the "ligand-binding domain" of the fusion protein encoded by the gene contained in the vector.

Any ligand can be used in the present invention as long as it acts on a specific protein to cause association of the protein, but a steroid hormone is preferable. Examples of the steroid hormone include estrogens, androgens, progesterone, glucocorticoids, and mineral corticoids. They are used in combination with their respective receptor proteins. Any cytokine receptor can also be used in the present invention as long as it imparts proliferation activity to a cell upon association. Examples of the cytokine receptor are those belonging to the cytokine receptor family including G-CSF and those belonging to the tyrosine kinase receptor family including c-kit and flk2/flt3.

As the "domain which imparts proliferation activity to a cell" of the fusion protein according to the present invention, it is possible to use a molecule that transmits the intracellular proliferation signal, for example, an entire molecule of a cytokine receptor. It is also possible to use only a domain in the molecule that imparts proliferating activity to a cell. The latter approach is advantageous in proliferating the cell as it is because the domain proliferates the cell into which the fusion protein-coding gene has been introduced without differentiating it. Furthermore, the vector used in the present invention includes not only a single vector molecule containing the fusion protein-coding gene and a single vector molecule containing the fusion protein- coding gene and the exogenous gene, but also includes a vector system of multiple vector molecules comprising a combination of a vector containing the fusion protein-coding gene and a vector containing the exogenous gene, for example, a binary vector system. Such a vector system of multiple vector molecules is usually introduced into a cell by co-transformation.

When a gene encoding the fusion protein and an exogenous gene are inserted into the same vector, they may be made into a dicistronic form containing an internal ribosome entry site (IRES) (published PCT Application in Japan No. Hei 6-509713). For example, it is possible to use a vector having a structure containing, from 5' to 3', a promoter, an exogenous gene, IRES, and a gene encoding the fusion protein or a vector having a structure containing, from 5' to 3', a promoter, a gene encoding the fusion protein, IRES, and an exogenous gene. The former type is generally used to allow most of the cells expressing the fusion protein gene to express the exogenous gene.

Moreover, in the present invention, the cell into which the vector is introduced includes hematopoietic stem cells, lymphatic cells, and cells other than these blood cells. In particular, hematopoietic stem cells that can self-proliferate are preferable in the present invention. Although the exogenous gene to be introduced into the cell in the present invention is not particularly limited, a normal, gene corresponding to a defective gene is generally used in the field of gene therapy.

### Brief Description of the Drawings

Fig. 1 (A) shows a chimeric molecule between the G-CSF receptor and the estrogen receptor (GCRER). (B) shows a mutant of the chimeric molecule between the G-CSF receptor and the estrogen receptor, deficient in the 5th through the 195th amino acids of the G-CSF receptor (GCR Δ (5-195)/ER). (C) shows a mutant of the chimeric molecule between the G-CSF receptor and the estrogen receptor, deficient in the 5th through 195th amino acids and the 725th through 756th amino acids of the G-CSF receptor (GCR Δ (5-195, 725-756)/ER).
Fig. 2 shows a retrovirus vector "pMX" in which a chimeric gene between the G-CSF receptor and the estrogen receptor has been incorporated.
Fig. 3 shows proliferation of the Ba/F3 cells transformed with "pCMX-GCRER" with the passage of time.
Fig. 4 shows proliferation of the Ba/F3 cells transformed with "pCMX-GCRER" with the passage of time; the cells were stimulated with various concentrations of estradiol.
Fig. 5 shows proliferation of the Ba/F3 cells transformed with "pCMX-GCRΔ(5-195)/ER" with the passage of time.
Fig. 6 shows plasmid "pCMX-GCRER-IRES-CD24."
Fig. 7 shows plasmid "pCMX-GCRΔ(5-195)/ER-IRES-CD24."
Fig. 8 shows plasmid "pCMX-GCRΔ(5-195, 725-756)/ER-IRES-CD24."
Fig. 9 shows the expression of CD24 in the Ba/F3 cells into which "pCMX-GCRΔ(5-195)/ER-IRES-CD24" has been introduced, detected by flow cytometry. The upper panel shows the results from the Ba/F3 cells into which "pCMX-GCR Δ (5-195)/ER-IRES-CD24" has been introduced; the lower panel shows the result from the Ba/F3 cells into which "pCMX-GCRΔ (5-195) /ER" has been introduced as a control. (Note that the data also contain the signal from propidium iodide that was used to detect dead cells.)
Fig. 10 is a microscopic photograph showing granulocyte-macrophage lineage colonies derived from bone marrow cells into which "vMXGCRER" has been introduced.
Fig. 11 is a microscopic photograph showing erythroblastic colonies derived from the bone marrow cells into which "vMXGCR Δ (5-195)/ER" has been introduced.
Fig. 12 is a microscopic photograph showing the Wright-Giemsa-stained macrophages which have differentiated from the bone marrow cells into which "vMXGCRER" was introduced.
Fig. 13 is a microscopic photograph showing the Wright-Giemsa-stained erythroblasts which have differentiated from the bone marrow cells into which "vMXGCRΔ(5-195)/ER" was introduced.

### Best Mode for Implementing the Invention

### Example 1 Constructing the chimeric G-CSF receptor/estrogen receptor gene (a selective amplification gene)

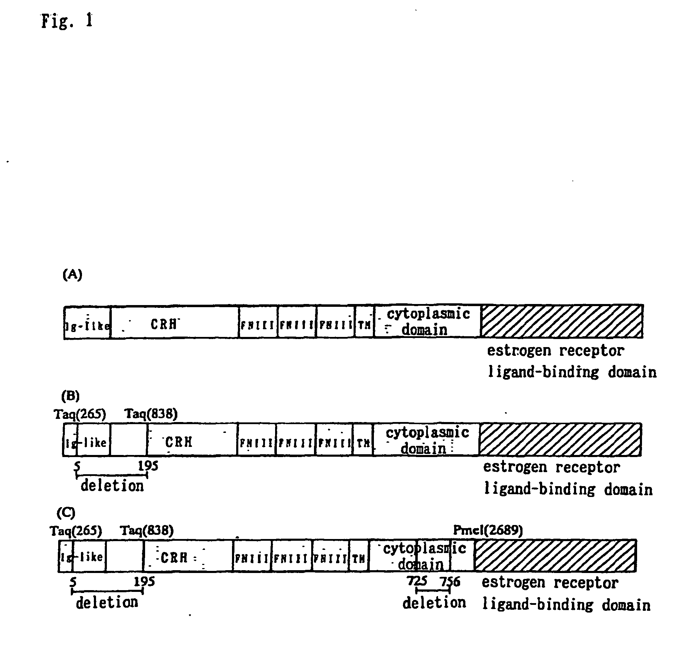

In order to produce a chimeric protein comprising the entire G-CSF receptor and the ligand (estrogen)-binding domain of the estrogen receptor (hereafter designated simply as "GCRER"), the fusion gene having cDNAs that encode the respective proteins (Fig. 1 (A)) was constructed. Next, a mutant of the fusion gene, "GCRER," which is deficient in the 5th residue, Glu, through the 195th residue, Leu, of the G-CSF receptor extracellular domain (hereafter designated simply as "GCRΔ(5-195)/ER") was consturcted, in order to produce a chimeric protein that lacks reactivity against G-CSF (Fig. 1(B)). Further, a mutant was constructed by deleting a portion containing the differentiation-inducing domain (725-756) of the G-CSF receptor from the mutant (hereafter designated simply as "GCRΔ (5-195, 725-756)/ER") (Fig. 1(C)).

### Example 2 Isolation of Ba/F3 cells into which was introduced the chimeric G-CSF receptor/estrogen receptor gene, which is a selective amplification gene

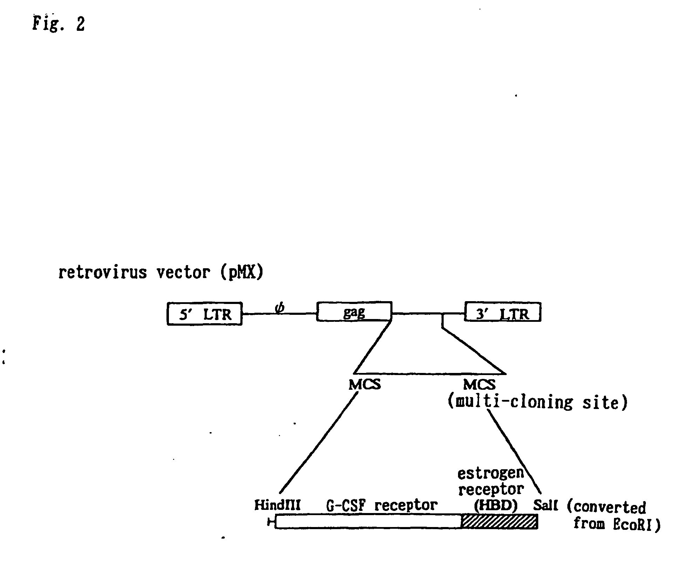

The three kinds of selective amplification genes prepared in Example 1 were introduced into plasmid "pCMX" (Cancer Res. 56:4164 (1996)). Ten *µ*g each of the resulting plasmids were introduced into the Ba/F3 cell, which is an IL-3-dependent cell line, together with 1 *µ* g of the ScaI-linearized "pSV2bsr" (Kaken Pharmaceuticals) carrying a blasticidin resistance gene, by electroporation. After the electroporation, the cells were distributed into 24-well plates at 5 x 10⁵ cells per well, and cultured in a medium containing 10 µg/ml of blasticidin. Proliferation of blasticidin resistant cells was observed in 11 out of 17 wells where "pCMX-GCRER" was introduced, in 3 out of 29 wells where "pCMX-GCRΔ (5-195) /ER" was introduced, and in 52 out of 52 wells where "pCMX-GCRΔ (5-195, 725-756)/ER" was introduced. After allowing these blasticidin resistant cells to proliferate in individual wells with IL-3, the cells were cultured with 10⁻⁷ M estradiol instead of IL-3. Proliferation of IL-3-independent and estrogen-dependent cells was observed in 7 out of 11 wells where "pCMX-GCRER" was introduced, in 3 out of 3 wells where "PCMX-GCRΔ(5-195)/ER" was introduced, and in 13 out of 16 wells where "pCMX-GCRΔ(5-195, 725-756)/ER" was introduced. When a similar experiment was performed using, in place of "pCMX-GCRER," a retrovirus vector "pMX" (Exp. Hematol. 24: 324 (1996)) into which "GCRER" had been inserted (hereafter designated simply as "pMX-GCRER") (Fig. 2), proliferation of IL-3-independent and estrogen-dependent cells was observed in 2 out of the 24 wells each containing one cell. Also, when 1 nM G-CSF was added in place of estradiol to the cells into which "pCMX-GCRER" was introduced, those wells that showed G-CSF-dependent proliferation were the same as those that had shown estradiol-dependent proliferation. Moreover, when the Ba/F3 cells containing no plasmid were used as a control, neither G-CSF-dependent proliferation nor estradiol-dependent proliferation was observed. The production of the desired fusion protein in the cells was confirmed by western blotting using an anti-G-CSF receptor antibody or an anti-estrogen receptor antibody.

### Example 3 Analysis of cell proliferation by estradiol

Among the clones obtained by limiting dilution in Example 2, those showing good response to estradiol were selected and used in the following experiment (XTT assay).

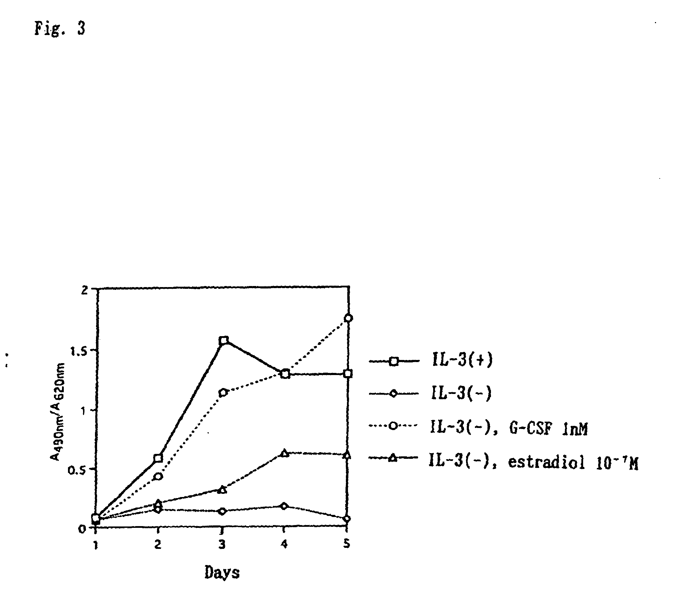

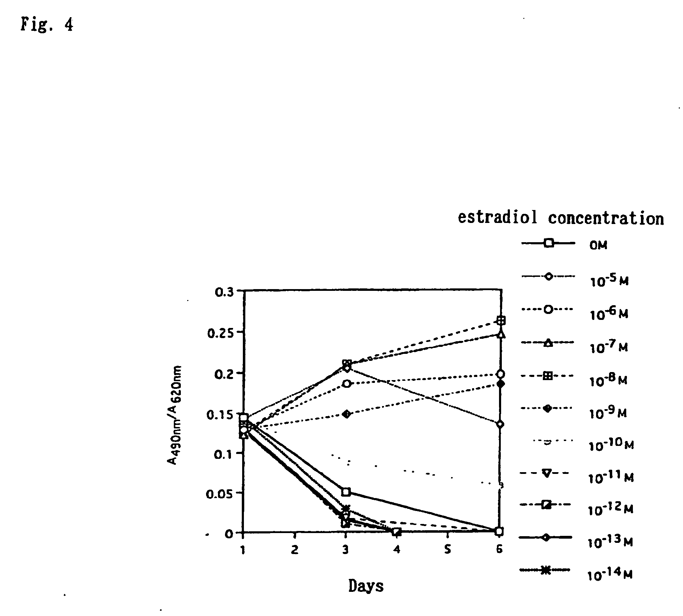

The Ba/F3 cells into which "pCMX-GCRER" had been introduced were examined. There were IL-3-independent cells that proliferated by stimulation with G-CSF or estradiol (Fig. 3). Moreover, when the same experiment was done while varying the estradiol concentrations between 10⁻¹⁴ and 10⁻⁷ M, cell proliferation was observed in the range from 10⁻⁹ to 10⁻⁷ M (Fig. 4). This result suggests that estradiol transmits the cell proliferation signal at the concentrations between 10⁻⁹ and 10⁻⁷ M.

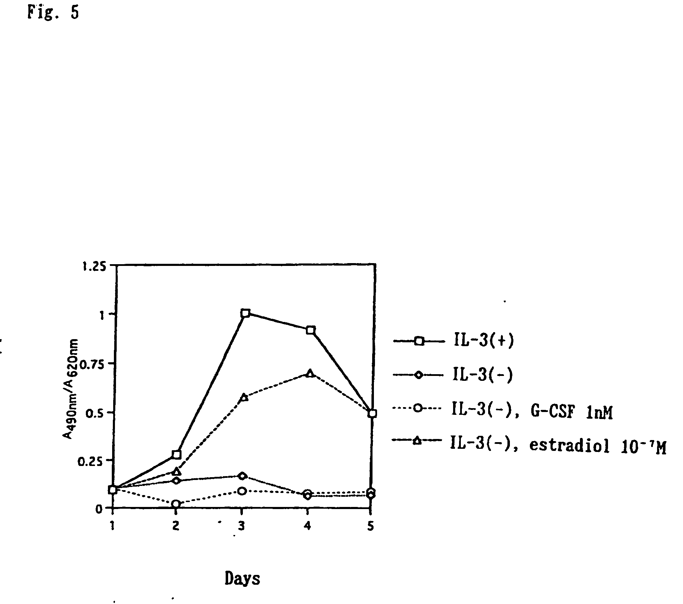

The Ba/F3 cells into which "pCMX-GCR Δ (5-195)/ER" had been introduced were then examined. The results indicated that the cell proliferation by G-CSF stimulation was blocked and the estradiol stimulation alone caused cell proliferation (Fig. 5).

Similarly, for Ba/F3 cells into which "pCMX-GR Δ (5-195, 725-756)/ER" had been introduced, cell proliferation was caused by estrogen stimulation, but no response to G-CSF was observed.

### Example 4 Construction of the IRES-CD24 expression plasmid

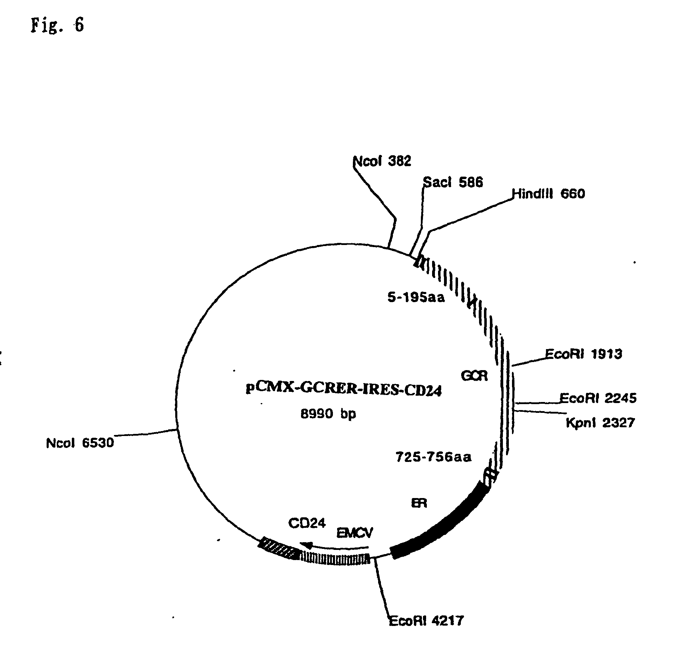

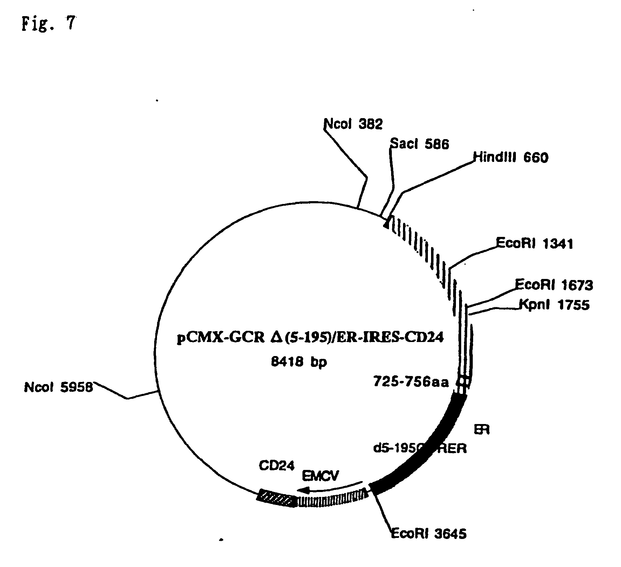

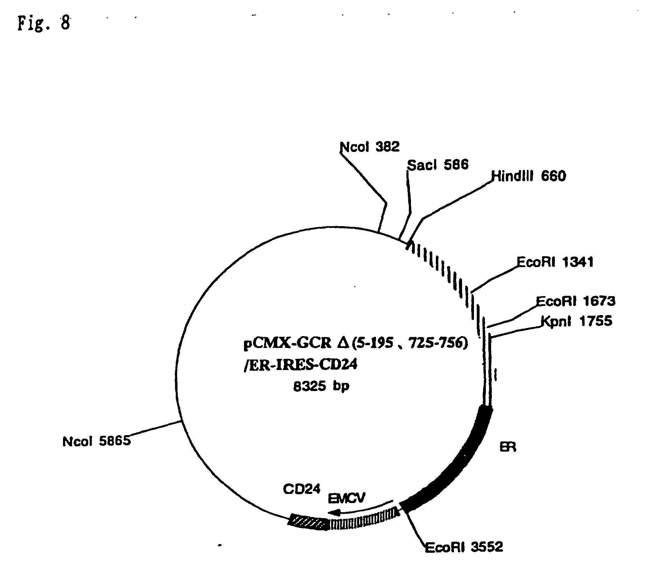

"PCMX-GCRER" was digested with HindIII and EcoRI, and the vector fragment ("fragment 1") was recovered. Also, from "pCMX-GCRER" and "pCMX-GCRΔ(5-195)/ER," the HindIII fragment ("fragment 2," 1672 bp) and the KpnI fragment ("fragment 3, "1099 bp), and the EcoRI fragment ("fragment 4," 1888 bp) and the KpnI fragment ("fragment 5," 1792 bp) were recovered. PBCEC (pBluescript II KS ligated to IRES and CD24 derived from EMCV, Migita, M., Proc. Natl. Acad. Sci. USA 92:12075 (1995)) was digested with ApoI, and the fragment containing IRES-CD24 ("fragment 6," 950 bp) was recovered. "pCMX-GCRER-IRES-CD24" (Fig. 6) was constructed by ligating "fragment 1," "fragment 2 , " "fragment 4," and "fragment 6." "pCMX-GCR△(5-195)/ER-IRES-CD24" (Fig. 7) was constructed by ligating "fragment 1, " "fragment 3," "fragment 4," and "fragment 6." "pCMX-GCR△(5-195, 725-756)/ER-IRES-CD24" (Fig. 8) was constructed by ligating "fragment 1," "fragment 3," "fragment 5," and "fragment 6."

### Example 5 Intracellular expression of CD24

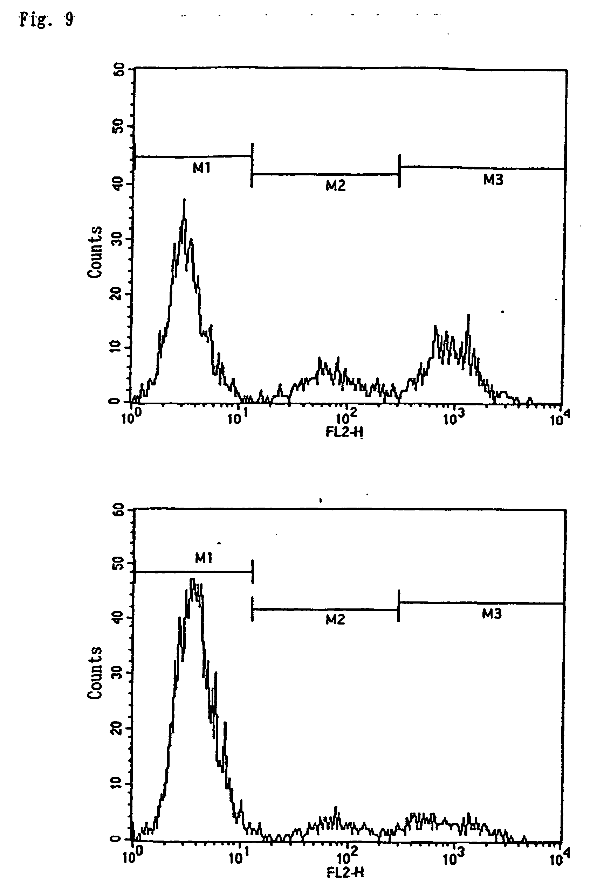

After 10⁷ Ba/F3 cells had been washed twice with PBS and once with "OPTI-MEM1" (Gibco-BRL), the cells were suspended into 0.2 ml of "OPTI-MEM1." Ten mg each of "pCMX-GCRER-IRES-CD24," "pCMX-GCR Δ (5-195)/ER-IRES-CD24;" and "pMX-GCR Δ (5-195, 725-756)/ER-IRES-CD24" was added to the cells, and transformation was performed using "Gene Pulser" (BioRad) at 290 V, 960 mF. After the transformation, the cells were cultured for two days in the RPMI medium containing 10% FCS and 10 U/ml mIL-3 (R&D SYSTEMS). After 10⁶ cells had been washed with 5 % FCS/PBS, the cells were reacted with 1 mg/ml of the anti-CD24 antibody (Pharmingen) for 30 minutes at room temperature. The cells were then washed twice with 5% FCS/PBS, reacted with a 1:20 dilution of the PE-labeled anti-mouse antibody (DAKO) for 30 minutes at room temperature, and washed again twice with 5% FCS/PBS. The cells were suspended in 1 ml of 5 mg/ml propidium iodide/PBS, and the CD24 expression was analyzed by flow cytometry (Becton Dickinson) using a 585 nm detector. The CD24 expression was detected from a number of the cells into which "pCMX-GCR△ (5-195) /ER-IRES-CD24" had been introduced. In this experiment, the cells into which "pCMX-GCR△ (5-195)/ER" had been introduced were used as a control against the cells having "pCMX-GCR△ (5-195) /ER-IRES-CD24" introduced. The results are shown in Fig. 9 and Table 1. Note that the data contain the signal from propidium iodide that was used to detect the dead cells.

**Table 1**

| Introduced plasmid | Anti-CD24 antibody (-) cells | Anti-CD24 antibody (+) cells |
|---|---|---|
| PCMX-GCRΔ (5-195) /ER-IRES-CD24 | 59.77 % | 40.23% |
| pCMX-GCRΔ (5-195) /ER | 85.10 % | 14.90 % |

### Example 6 Progenitor assays

5-Fluorouracil (5FU: Wako Pure Chemical Industries, Ltd.) in physiological saline (10 mg/ml) was intravenously injected into four 6-week-old C57BL mice at a dose of 330 ml/mouse. Two days after the injection, bone marrow was collected from femurs, centrifuged (1,500 rpm, 25°C , 22 min) on "Lymphocyte-M" (Cederlane) to isolate mononuclear cells. The mononuclear cells were cultured for two days in the Iscove modified Dulbecco medium (IMDM; Gibco) supplemented with 20% FCS, 100 U/ml IL6, and 100 mg/ml rat SCF. On a CH296 (Takara Shuzo; Hanenberg, H. et al., Nature Med. 2: 876 (1996))-coated plate (1146: Falcon) 10⁶ bone marrow cells pretreated with IL6 and SCF were suspended in a culture supernatant containing 10⁸ of either the retrovirus "vMXGCRER" (obtained in the culture supernatant of an ecotropic packaging cell line "GP+E-86" (J. Virol. 62: 1120 (1988)) and having "pMX-GCRER" incorporated therein) or the retrovirus "vMXGCR△ (5-195)/ER" (obtained in the culture supernatant of an ecotropic packaging cell line "GP+E-86" and having "pMX-GCR△(5-195)/ER" introduced therein). The cells were cultured in the presence of IL6 and SCF. The viral supernatants were replaced at 2, 24, 26, 36, and 38 hours. Twenty-four hours after the sixth viral supernatant replacement, the cells were transferred into a medium containing methylcellulose (IMDM, 1.2% methylcellulose 1,500 cp; Wako, 20% FCS, 1% deionized BSA, 10 mM 2-mercaptoethanol, 10⁻⁷ M b-estradiol) at 10'/well. After culturing for 10 days, colonies were observed under the microscope. Smear samples were prepared and subjected to Wright-Giemsa staining to identify the cells.

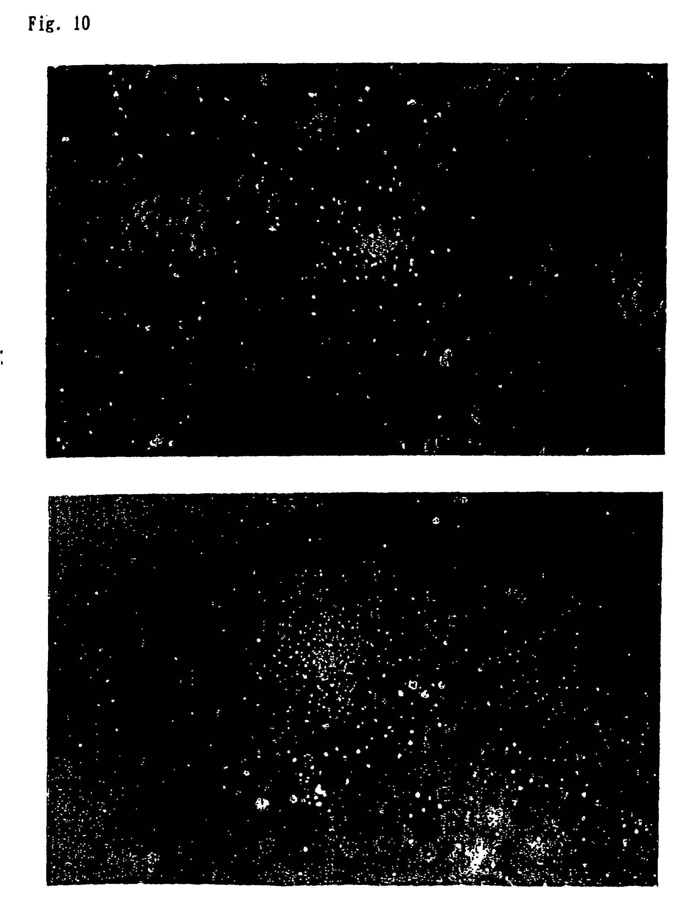

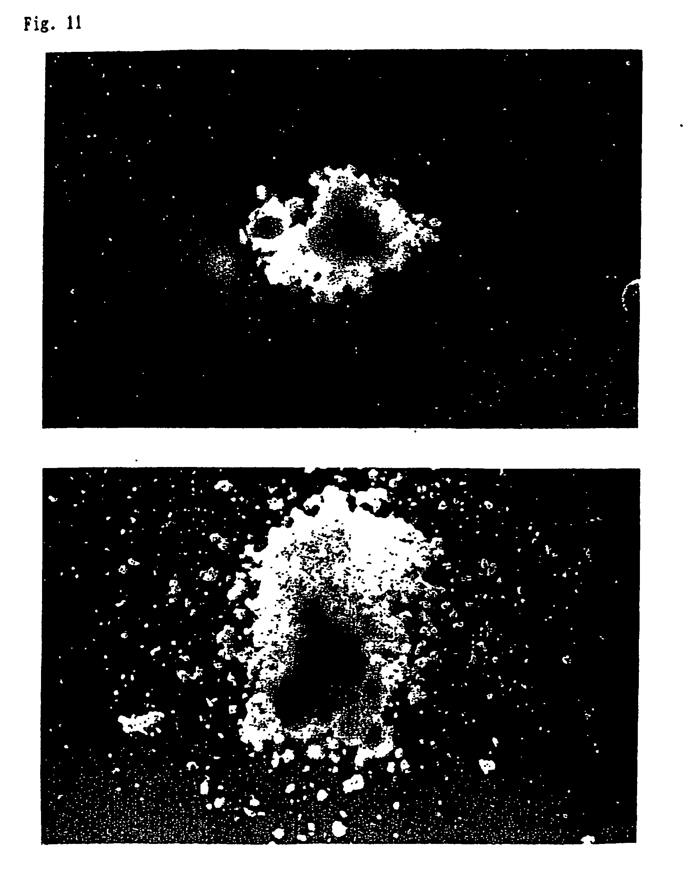

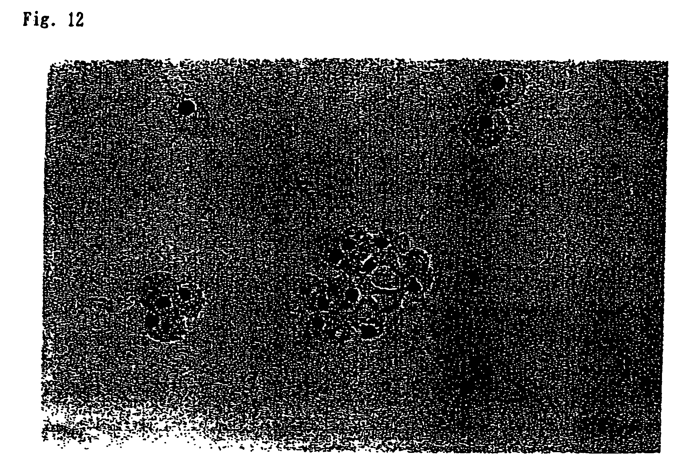

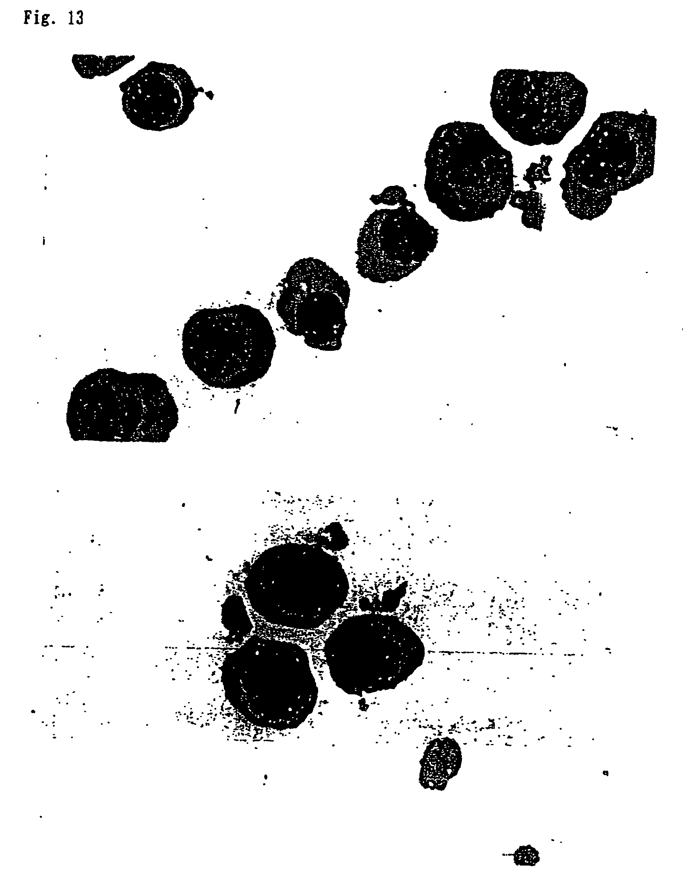

Among the bone marrow cells infected with "vMXGCRER" or "vMXGCR Δ (5-195)/ER," granulocyte-macrophage lineage colonies and erythroblast lineage colonies, which had differentiated from the bone marrow cells by the estradiol stimulation, were observed. Fig. 10 shows the granulocyte-macrophage lineage colonies derived from the "vMXGCRER"-infected bone marrow cells by the estradiol stimulation; Fig. 11 shows the erythroblast lineage colonies derived from the "vMXGCRΔ(5-195)/ER"-infected bone marrow cells upon the estradiol stimulation. When the cells constituting these colonies were made into smear samples and subjected to wright-Giemsa staining, differentiated blood cell images were obtained. Fig. 12 shows the Wright-Giemsa stained image of the macrophage observed in the smear samples of the granulocyte-macrophages lineage colonies derived from the "vMXGCRER"-infected bone marrow cells; Fig. 13 shows the Wright-Giemsa stained image of the erythroblasts observed in the smear samples of the erythroblast lineage colonies derived from the "vMXGCR Δ(5-195)/ER"-infected bone marrow cells.

### Industrial Applicability

The present invention has made it possible to selectively amplify a cell into which an exogenous gene has been introduced, in response to an external stimulus, thereby enabling effective gene therapy even when the introduction efficiency of the gene into the target cells is low. Furthermore, since the system for selectively amplifying cells of the present invention can be applied to various blood cells, the range of cells targeted in gene therapy has been widened. Therefore, the present invention provides an important basic technology, particularly in the field of gene therapy.

## Claims

1. A fusion protein comprising
(a) a first polypeptide and
(b) a second polypeptide,
wherein said first polypeptide comprises a ligand-binding domain of a steroid hormone receptor that self-associates upon ligand binding, and wherein said second polypeptide comprises a cytokine receptor or a proliferation-inducing part thereof that, upon said self-association of said first polypeptide, imparts proliferation activity to a cell.

2. The fusion protein of (1), wherein the steroid hormone receptor is an estrogen receptor.

3. The fusion protein of (1), wherein the cytokine receptor is a G-CSF receptor.

4. A vector comprising a gene encoding the fusion protein of (1).

5. A cell carrying the vector of (4).

6. An in vitro method for selectively proliferating the cell of 5 which comprises exposing the cell of (5) to a ligand capable of acting on the "ligand-binding domain" of the fusion protein of (1).

7. A vector comprising a desired exogenous gene and a gene encoding the fusion protein of (1).

8. The vector of (7), wherein the steroid hormone receptor is an estrogen receptor.

9. The vector of (7), wherein the cytokine receptor is a G-CSF receptor.

10. The vector of (7), wherein the "gene encoding a fusion protein" and the "exogenous gene" are located on the same molecule.

11. The vector of (7), wherein the "gene encoding a fusion protein" and the "exogenous gene" are located on separate molecules.

12. A cell carrying the vector according to any one of (7) to (11).

13. An in vitro method for selectively proliferating the cell of (12), which comprises exposing the cell of (12) to a ligand capable of acting on the "ligand-binding domain" of the fusion protein encoded by the gene contained in the vector of (7).

14. A kit comprising (a) the vector of (4) or (7) and (b) a ligand capable of acting on the "ligand-binding domain" of the fusion protein encoded by the gene contained in the vector.

## Patentansprüche

1. Fusionsprotein umfassend
(a) ein erstes Polypeptid und
(b) ein zweites Polypeptid,
wobei das erste Polypeptid eine Ligandenbindungsdomäne eines Steroidhormonrezeptors umfasst, der bei Ligandenbindung selbst-assoziiert und
wobei das zweite Polypeptid einen Cytokinrezeptor oder einen proliferationsinduzierenden Teil davon umfasst, der bei der Selbst-Assoziierung des ersten Polypeptids einer Zelle Proliferationsaktivität verleiht.

2. Fusionsprotein nach Anspruch 1, wobei der Steroidhormonrezeptor ein Östrogenrezeptor ist.

3. Fusionsprotein nach Anspruch 1, wobei der Cytokinrezeptor ein G-CSF-Rezeptor ist.

4. Vektor, der ein Gen umfasst, das das Fusionsprotein nach Anspruch 1 codiert.

5. Zelle, die den Vektor nach Anspruch 4 enthält.

6. *In vitro-* Verfahren zur selektiven Proliferierung der Zelle nach Anspruch 5, umfassend das Aussetzen der Zelle nach Anspruch 5 einem Liganden, der in der Lage ist, auf die "Ligandenbindungsdomäne" des Fusionsprotein nach Anspruch 1 zu wirken.

7. Vektor, der ein gewünschtes exogenes Gen und ein das Fusionsprotein nach Anspruch 1 codierendes Gen umfasst.

8. Vektor nach Anspruch 7, wobei der Steoridhormonrezeptor ein Östrogenrezeptor ist.

9. Vektor nach Anspruch 7, wobei der Cytokinrezeptor ein G-CSF-Rezeptor ist.

10. Vektor nach Anspruch 7, wobei das "Gen, das ein Fusionsprotein codiert" und das "exogene Gen" auf dem selben Molekül lokalisiert sind.

11. Vektor nach Anspruch 7, wobei das "Gen, das ein Fusionsprotein codiert" und das "exogene Gen" auf separaten Molekülen lokalisiert sind.

12. Zelle, die den Vektor nach einem der Ansprüche 7 bis 11 enthält.

13. *In vitro*-Verfahren zur selektiven Proliferierung der Zelle nach Anspruch 12, umfassend das Aussetzen der Zelle nach Anspruch 12 einem Liganden, der in der Lage ist, auf die "Ligandenbindungsdomäne" des Fusionsproteins zu wirken, das von dem Gen codiert wird, das in dem Vektor nach Anspruch 7 enthalten ist.

14. Kit umfassend (a) den Vektor nach Anspruch 4 oder 7 und (b) einen Liganden, der in der Lage ist, auf die "Ligandenbindungsdomäne" des Fusionsproteins zu wirken, das von dem Gen codiert wird, das auf dem Vektor enthalten ist.

## Revendications

1. Protéine de fusion comprenant
(a) un premier polypeptide et
(b) un second polypeptide,
dans laquelle ledit premier polypeptide comprend un domaine de liaison au ligand d'un récepteur d'hormone stéroïde qui s'auto-associe après la liaison du ligand, et dans laquelle le second polypeptide comprend un récepteur de cytokine ou une partie de celui-ci induisant la prolifération qui, après ladite auto-association dudit premier polypeptide, confère une activité de prolifération à une cellule.

2. Protéine de fusion selon (1), dans laquelle le récepteur d'hormone stéroïde est un récepteur d'oestrogène.

3. Protéine de fusion selon (1), dans laquelle le récepteur de cytokine est un récepteur de G-CSF.

4. Vecteur comprenant un gène codant la protéine de fusion selon (1).

5. Cellule portant le vecteur selon (4).

6. Procédé *in vitro* pour faire proliférer de manière sélective la cellule selon (5), qui comprend l'exposition de la cellule selon (5) à un ligand capable d'agir sur le « domaine de liaison au ligand » de la protéine de fusion selon (1).

7. Vecteur comprenant un gène exogène souhaité et un gène codant la protéine de fusion selon (1).

8. Vecteur selon (7), dans lequel le récepteur d'hormone stéroïde est un récepteur d'oestrogène.

9. Vecteur selon (7), dans lequel le récepteur de cytokine est un récepteur de G-CSF.

10. Vecteur selon (7), dans lequel le « gène codant une protéine de fusion » et le « gène exogène » sont localisés sur la même molécule.

11. Vecteur selon (7), dans lequel le « gène codant une protéine de fusion » et le « gène exogène » sont localisés sur des molécules différentes.

12. Cellule portant le vecteur selon l'une quelconque de (7) à (11).

13. Procédé *in vitro* pour faire proliférer de manière sélective la cellule selon (12), qui comprend l'exposition de la cellule selon (12) à un ligand capable d'agir sur le « domaine de liaison au ligand » de la protéine de fusion codée par le gène contenu dans le vecteur selon (7).

14. Kit comprenant (a) le vecteur selon (4) ou (7) et (b) un ligand capable d'agir sur le « domaine de liaison au ligand » de la protéine de fusion codée par le gène contenu dans le vecteur.
